**Europäisches Patentamt**

**European Patent Office**

**Office européen des brevets**

(19)

(11) Publication number : **0 369 164 B1**

(12)

# EUROPEAN PATENT SPECIFICATION

(45) Date of publication of patent specification :
**10.03.93 Bulletin 93/10**

(51) Int. Cl.⁵ : **C08L 53/02,** C08L 67/04,
A61M 5/315, // (C08L53/02,
67:02), (C08L67/02, 53:02)

(21) Application number : **89118883.1**

(22) Date of filing : **11.10.89**

(54) **Gasket and medical device using the same.**

(30) Priority : **18.10.88 JP 260624/88**

(43) Date of publication of application :
**23.05.90 Bulletin 90/21**

(45) Publication of the grant of the patent :
**10.03.93 Bulletin 93/10**

(84) Designated Contracting States :
**BE DE ES FR GB IT NL SE**

(56) References cited :
**CH-A- 648 759**
**GB-A- 2 122 627**

(73) Proprietor : **TERUMO KABUSHIKI KAISHA**
**No. 44-1, Hatagaya 2-chome, Shibuya-ku**
**Tokyo 151 (JP)**

(72) Inventor : **Akaike, Yoshiaki**
**Terumo K.K. 1727-1, Tsukijiarai, Showa-cho**
**Nakakoma-gun Yamanashi (JP)**

(74) Representative : **Casalonga, Axel et al**
**BUREAU D.A. CASALONGA - JOSSE**
**Morassistrasse 8**
**W-8000 München 5 (DE)**

EP 0 369 164 B1

Jouve, 18, rue Saint-Denis, 75001 PARIS

## Description

The present invention relates to a gasket and a medical device using the same.

There are medical devices such as disposable injectors each of which comprises a cylindrical body and a gasket which can move in closely contact with the inner surface of the cylindrical body.

Conventionally, the above-described gasket is made of, as disclosed in Japanese Patent Publication No. 59-18427, vulcanized rubber or styrene type thermoplastic elastomer (to be called "TPE" hereinafter). The styrene type TPE is made of a composite consisting of hydrogen-added derivative of styrene-butadiene block copolymer, softener for rubber, and polypropylene for improving workability and for adjusting the hardness.

However, it needs a large facility to manufacture the gasket made of the vulcanized rubber since such a gasket is manufactured after it has been subjected to a complicated vulcanization process. Furthermore, a variety of additives such as sulfur, a vulcanization accelerator, and a filler are necessary. As a result, there is a fear of an elution of the additives into the liquid drug when the medical device is used.

On the contrary, although the gasket made of the styrene TPE exhibits an advantage in the formability at the time of the injection molding and another advantage in that the fear of elution of sulfur can be eliminated, it is disadvantageous to vulcanized rubber in terms of the elasticity of the rubber, where the term "the elasticity of rubber" means in particularly in the tensile characteristics that the inflection points such as the yielding points are reduced as possible and also the modulus (for example, the stress when expanded by 300%) is at a relatively high level in the relationship with the hardness. That is, in the styrene TPE, polypropylene which is the component of it causes the inflection point such as the yield point to be generated and causes the modulus to be lowered in the tensile characteristics. Therefore, in a case where the gasket made of the styrene TPE is employed in an injector, the displacement added to a portion to be pushed does not immediately correspond to the distance of movement of the gasket. Therefore, a problem arises in that the gasket intermittently moves, that is, a so-called pulsation phenomenon takes place.

An object of the present invention is to provide a gasket and a medical device using the same, the gasket exhibiting an excellent formability and safety against elution and capable of preventing pulsation phenomenon.

A gasket in accordance with the present claimed invention is consisted essentially of a composition comprising hydrogen-added derivative (component $\underline{a}$) of styrene-butadiene block copolymer, softener for rubber (component $\underline{b}$), and polyester elastomer (component $\underline{c}$).

A gasket in accordance with the present claimed invention is that the composition is formed by mixing 20 to 150 parts by weight of the component $\underline{c}$ and 100 parts by weight consisting of the component $\underline{a}$ and the component $\underline{b}$, the 100 parts being the total of the component $\underline{a}$ by 30 to 70 wt% and the component $\underline{b}$ by 70 to 30 wt%.

A medical device in accordance with the present invention in claim 3 includes a cylindrical body and a gasket capable of moving in close contact with the inner surface of the cylindrical body, the medical device being characterized in that the gasket is the type according to the claims.

Then, the components and proportion of the mixture of the composite forming the gasket according to the present invention will be described in detail.

## Component a

The above-described component $\underline{a}$ according to the present invention is hydrogen-added derivative of block copolymer expressed by a general formula:

$$A\text{-}(B \text{ - } A)n \text{ or } A \text{ - } B\text{-}(A \text{ - } B)n$$

[where A represents a polymer block of monovinyl-substituted aromatic hydrocarbon, B represents a conjugate diene elastomer copolymer block, and n represents an integer ranged between 1 and 5].

Although a variety of materials can be exemplified as the monovinyl-substituted aromatic hydrocarbon composing the polymer block A, it is preferable to use styrene or $\alpha$-methyl styrene. It is preferable to use butadiene or isoprene as the conjugated diene monomer of the polymer block B in the formula. Alternatively, their mixture may be used. In a case where butadiene is used as sole conjugated diene monomer to form the polymer block B, it is preferable for the elastomer characteristics to be maintained that the polymerization conditions are employed under which the proportion of the 1, 2-micro structure in the micro structures in polybutadiene becomes 20 to 50%, further preferably 1, 2-micro structure is 35 to 45%. Furthermore, it is preferable that the proportion of the copolymer block B with respect to the above-described copolymer is at least 65 wt%.

It is preferable that the average molecular weight of the above-described polymer block A is ranged between 5,000 and 125,000. It is also preferable that the same of the block B is ranged between 15,000 and 250,000.

The block copolymer according to the present invention is the block copolymer which has been subjected to a hydrogen addition process.

Although a variety of methods of manufacturing the hydrogen-added derivative of the block copolymer expressed by the above-described general formula have been disclosed, typical methods have been disclosed in, for example, Japanese Patent Publication Nos. 42-8704 and 43-6636.

It is preferable that the addition of hydrogen in the manufacturing process for the block copolymer is conducted such that hydrogen is added at least 50%, preferably 80% or more of olefinic linkages in the polymer block B and hydrogen is also added to 25% or more of aromatic unsaturated bonds in the polymer block A. As the block polymer of this type, a polymer on the market such as KRATON-G (trade name of polymer manufactured by Shell Chemical) can be used.

## Component b

It is preferable that a mineral oil type or synthetic resin softener is used as the component b which is the softener for rubber according to the present invention.

The mineral type softener is a mixture consisting of aromatic rings, naphthene rings, and paraffin chains. The softener is classified such that the mixture in which the number of carbons in the paraffin chains exceeds 50% is called a paraffin type softener, the mixture in which the same in the naphthene rings is ranged between 30 and 45% is called a naphthene type softener, and the mixture in which the number of the aromatic carbons exceeds 30% is called an aromatic type softener. The preferable mineral type softeners as the component b of the present invention are the naphthene type softener and the paraffin type softener in which the proportion of the aromatic hydrocarbon thereof is less than 30%. The aromatic softer is not preferably used in terms of dispersion characteristics with respect to the above-described component a according to the present invention.

The properties of the mineral type softer are arranged such that the coefficient of kinetic viscosity at 37.8°C is 20 to 500 $10^{-6}m^2/s$ (cst), the pour point is -10 to -15°C, and the flash point (COC) is 170 to 300 °C.

Although polybutene, low molecular weight polybutadiene, or the like can be used, the mineral type softener is preferably used.

## Component C

The following material can be used as the polyester type elastomer which serves as the component c according to the present invention.

① block copolymer consisting of an aromatic polyester block (a) and a non-aromatic polyester block (b)

② block copolymer consisting of an aromatic polyester block (a) and a polyether block (c)

③ block copolymer consisting of an aromatic polyester block (a) and the above-described components (b) and (c)

The aromatic polyester block (a) is made of polyester oligomer prepared by condensing aromatic dicarboxylic acid and diol, it being exemplified by polyethylene terephthalate oligomer, polypropylene terephtalate oligomer, polytetramethylene terephthalate oligomer, and polypentamethylene terephthalate oligomer.

The non-aromatic polyester block (b) is made of either of the following materials:

(1) polyester oligomer prepared by condensing aliphatic or alicyclic caroxylic acid and aliphatic diol

(2) polyester oligomer synthesized from aliphatic lactone or aliphatic monool carboxylic acid

The former polyester oligomer (1) is exemplified by polyester oligomer prepared by condensing at least a material selected from a group consisting of alicyclic dicarboxylic acid such as 1, 4-cyclohexane dicarboxylic acid and 1, 2-cyclohexane dicarboxylic acid, dicyclohexyl-4, 4'-dicarboxylic acid and aliphatic dicarboxylic acid such as succinic acid, oxalic acid, adipic acid, and sebacic acid and at least one of diols such as ethylene glycol, propylene glycol, tetramethylene glycol, and pentamethylene glycol. The latter polyester oligomer (2) is exemplified by polycaprolactone polyester oligomer synthesized from ε-caprolactone or ω-oxycaproic acid.

The polyether block (c) is exemplified by polyether oligomer of the average molecular weight of about 400 to 6,000 such as poly (alkylene oxide) glycol.

The poly (alkylene oxide) glycol is exemplified by the block or random copolymer of polyethylene glycol, poly (1, 2 and 1, 3 propylene oxide) glycol, poly (tetramethylene oxide) glycol, poly (hexamethylene oxide) glycol, ethylene oxide, and propylene oxide, or the block or the random copolymer of ethylene oxide and tetrahydrofuran.

The polyester elastomer (the component c) may be manufactured by previously synthesizing the blocks of the oligomer composed by the components thereof respectively, and by condensing between the blocks by way of the ester bonding. It may be manufactured by another method which is arranged such that the block (b) is previously polymerized and then it is mixed with the component monomer of the block (a).

As the polyester elastomer of the type described above, Peruprene P, S (trade name of Toyobo) or Hitolel (trade name of Tore. Dupont) on the market can be used.

In general softener for rubber and a resin component (for example, polypropylene) for adjusting the hardness and the workability are added to thermoplastic elastomer whose base is made of hydrogen-added derivative of styrene-budadiene block copolymer. The present invention is characterized in that polyester elastomer is, as the resin component, added by a certain quantity.

That is, it has been considered conventionally that thermoplastic elastomer exhibiting heat resistance and rubber elasticity can be obtained by using polypropylene resin. However, the inventors found that the rubber elasticity and the heat resistance can be significantly improved by using polyester elastomer in replacement of the polypropylene resin.

Mixture and Kneading

It is preferable that the mixture ratio of the components $\underline{a}$, $\underline{b}$, and $\underline{c}$ in the thermoplastic elastomer according to the present invention is arranged such that: the component $\underline{a}$ is 30 to 70 wt%, preferably, 35 to 60 wt%. If it is less than 30 wt%, the rubber elasticity and the heat resistance of the obtained thermoplastic elastomer are insufficient. If the same exceeds 70 wt%, the flexibility, formability and workability deteriorate.

The component $\underline{b}$ is arranged to be 70 to 30 wt%, preferably 65 to 40 wt%. If it exceeds 70 wt%, the rubber elasticity and the heat resistance are insufficient. If the same is less than 30 wt%, the flexibility, formability, and workability deteriorate.

The component $\underline{c}$ is arranged to be 20 to 150 parts by weight with respect to 100 parts which is the total of the components $\underline{a}$ and $\underline{b}$, preferably 30 to 100 parts by weight. If it is less than 20 parts by weight, the formability and workability deteriorate, while the same exceeds 150 parts by weight, the flexibility is lost.

Although the present invention is characterized in that the necessary components are contained by a certain quantity when thermoplastic elastomer is obtained, polyolefine polymer, polystyrene polymer, and inorganic filler may be added in a range with which the basic performance of the invention does not deteriorate, the inorganic filler being such as calcium carbonate, talc, mica, and carbon black. Furthermore, in order to meet the aiming property, additives such as an antioxidant, ultraviolet absorber, slipping agent, and fluidity accelerator (for example, polyethylene wax) may be mixed.

The thermoplastic elastomer according to the present invention can be manufactured by the mechanical melt kneading. Specifically, usual mechanical melt kneader such as a Banbury mixer, various kneaders, single or two-shaft extruder can be used.

The thermoplastic elastomer used in the present invention may be formed by the method of molding thermoplastic resin such as the injection molding, extrusion molding, and blow molding.

That is, the TPE forming the gasket according to the present invention is able to cause for the rubber elasticity involved by the hydrogen-added derivative of styrene-butadiene block copolymer to be maintained and also to cause for the formability and safety against elution to be secured by way of the addition of polyester elastomer as an alternative to polypropylene which forms the above-described conventional polypropylene. As a result, a gasket and a medical device using the same which exhibits an excellent formability and safety against solution without any problem of pulsation phenomenon can be obtained.

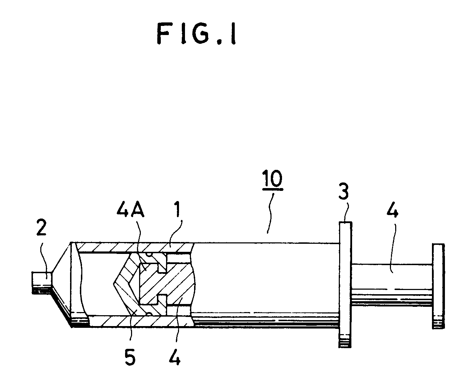

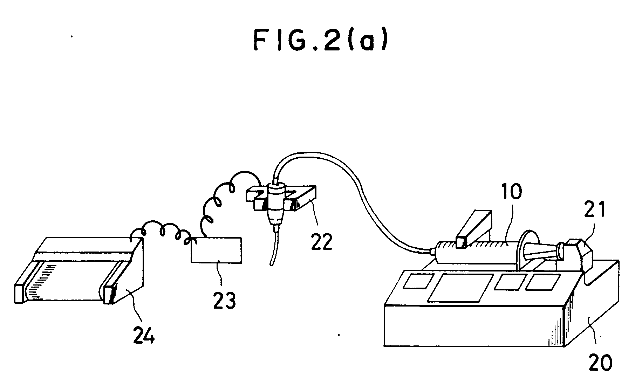

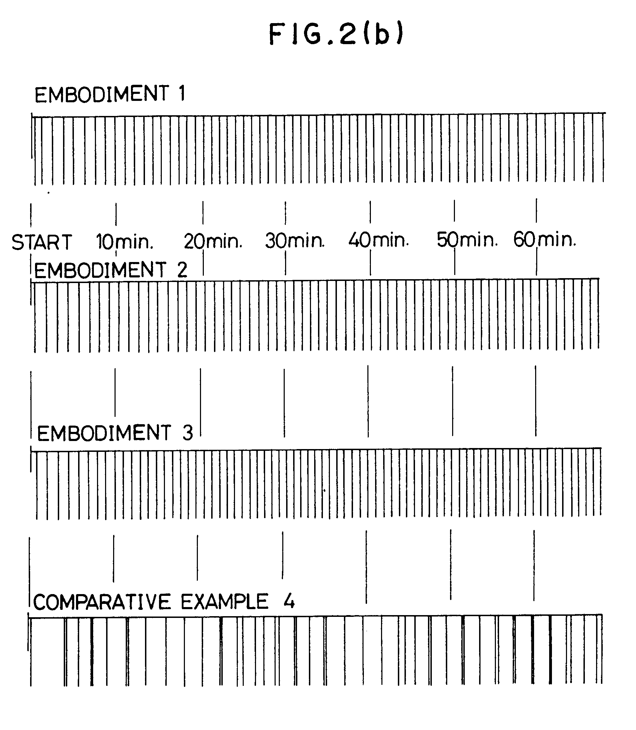

Fig. 1 is a schematic view of an injector in which the gasket according to the present invention is used, and Fig. 2 is a diagram which illustrates the results of a pulsation test.

In an injector 10 shown in Fig. 1, reference numeral 1 represents a syringe made of hard synthetic resin, 2 represents a nozzle, and 3 represents a flange. Reference numeral 4 represents a body to be pushed which is made of hard synthetic resin, and 5 represents a gasket according to the present invention which is fitted to a front portion 4A to be engaged of the body 4 to be pushed.

In the following embodiments and comparative examples, the testing methods for making various evaluation were as follows, where test sample was a 2mm-thick sheet (sample for the following tests (1) and (2)) manufactured by a 141.75 g (5-ounce) in-line screw type injection molding machine under the conditions that the injection pressure was 500 kgf/cm$^2$, injection temperature was 220 °C, and the temperature of the mold was 40 °C. Furthermore, another test sample was a gasket for 5ml syringe having the peak diameter of 13.2mm (sample for the following tests (3) to (5)). The inner diameter of the outer cylinder for 5ml-syringe was 13.0mm.

(1) JIS-A hardness...JISK-6301

If the gasket for a cylinder is too hard, the sliding ability is insufficient and the assembling of it is difficult. On the contrary, if it is too soft, it cannot be strongly engaged to the body to be pushed, causing the separation of them to occur. It is preferable that the hardness is ranged between 20 or more and 85 or less.

(2) Stress when compressed by 300% (kgf/cm$^2$) ...JISK-6301

In the relationship with hardness, the higher the stress when compressed by 300% becomes, the pulsation can be prevented. When the stress when compressed by 300% becomes 22 to 25 kgf/cm$^2$ under the condition that the hardness is 68 to 65, the pulsation is caused to occur.

(3) Formability

The flowing characteristics at the time of conducting the injection molding were evaluated.

(4) Pulsation

A syringe pump 20 as shown in Fig.2 which is capable of pushing at a constant speed the body to be pushed which had been inserted into the syringe was used. The syringe 1 which had sucked water was engaged to the syringe pump 20. The quantity of injection of water from the syringe by the pushing head 21 of the syringe pump 20 was determined to be 1 ml/hour. The quantity of water injected from the syringe 1 was detected as a water drop of about 1/60ml by a detector 22. The detected results were recorded by a recording device 24 via an amplifier 23 so that the diagram about the signal was obtained as shown in Fig. 2. If the intervals between the signal shown in Fig. 2(b) are constant, it can be said that the quantity of injection per unit time period is constant and thereby there is no pulsation.

(5) Oil Bleed

The elution level of the softener for rubber was tested. It was evaluated in such manner that the state of the surface of the gasket at 23 °C for 7 days continued was visually observed.

The mixed components used in the embodiments and comparative examples were as follows:

(a) Hydrogen added derivative of styrene-budatiene block copolymer..."KRATON-G1651" manufactured by Shell Chemical (the Brookfield viscosity: 2 Pa.s 25°C (2000cps. 77° F) in 20 wt% toluene solution)

(b) Softener for rubber..."PW380" fluid paraffin manufactured by Idemitsu Kosan (kinetic viscosity at 40 °C: 381.6 $10^{-6}$m$^2$/s (cst))

(c-1) Polyester elastomer..."P-70B" peruprene (polyester ether elastomer) manufactured by Toyobo (JIS-A code 96 [-], melting point of the crystal: 200 °C)

(c-2) Polyester elastomer... "P-30B" (JIS-A code 70 [-], melting point of the crystal: 160 °C)

(c-3) Polyester elastomer..."P-150B" peruprene (polyester ether elastomer) manufactured by Toyobo (JIS-A code 98 [-], melting point of the crystal: 212 °C)

(c-4) Polyproplylene resin for comparative example..."Mistubishi Polypro BC5C" (MFR: 5g/10 minutes)

Embodiments 1 to 3 and Comparative Example 4

Phenol antioxidant "Iruganox 1010" was added by 0.1 parts by weight as a stabilizer to the mixtures shown in Table 1 with respect to 100 parts of the mixtures. Then, it was fused and kneaded by a two-shaft extruder of L/D = 33 and the cylinder diameter of 45 mm at predetermined temperature 220°C so that TPE pellet was obtained.

The 2mm-sheet to be tested and the gasket to be tested and having the peak diameter of 13.2mm were manufactured by the above-described injection molding in which the pellet described above was used. The results of the various evaluations about the obtained sheets and gaskets are shown in Table 1.

The gaskets according to the embodiments 1 to 3 are the gaskets claimed in claims 1 and 2, the gasket displaying an excellent stress at the same hardness and satisfactory rubber elasticity. In addition, the results of the testing about the formability, the pulsation, and oil bleed were satisfactory.

Table 1

| | | | Embodiments | | | Comparative Example |
|---|---|---|---|---|---|---|
| | | | 1 | 2 | 3 | 4 |
| Mixture (wt%) | | a | 100 | 100 | 100 | 100 |
| | | b | 70 | 70 | 70 | 70 |
| | | c-1 | 100 | | | |
| | | c-2 | | 40 | | |
| | | c-3 | | | 70 | |
| | | c-4 | | | | 35 |
| JIS-A hardness | | | 65 | 40 | 68 | 65 |
| 300% stress (kgf/cm$^2$) | | | 41 | 18 | 46 | 25 |
| Formability at molding | | | G | G | G | G |
| Pulsation | | | G | G | G | NG |
| Oil bleed | | | G | G | G | G |

## Claims

1. A gasket consisting essentially of a composition comprising hydrogen-added derivative (component $\underline{a}$) of styrene-butadiene block copolymer expressed by the general formula A-(B-A)$_n$ or A-B-(A-B)$_n$ where A represents a polymer block of monovinyl-substituted aromatic hydrocarbon, B represents a conjugate diene elastomer copolymer block and n represents an integer ranged between 1 and 5, softener for rubber (component $\underline{b}$), and polyester elastomer (component $\underline{c}$), the component $\underline{c}$ being arranged to be 20 to 150 parts by weight with respect to 100 parts by weight which is the total of the components $\underline{a}$ and $\underline{b}$.

2. A gasket according to claim 1, wherein said composition is formed by mixing 20 to 150 parts by weight of said component $\underline{c}$ and 100 parts by weight consisting of said component $\underline{a}$ and said component $\underline{b}$, said 100 parts being the total of said component $\underline{a}$ by 30 to 70 wt% and said component $\underline{b}$ by 70 to 30 wt%.

3. A gasket according to claim 1 or 2, wherein said component $\underline{c}$ is arranged to be 30 to 100 parts by weight with respect to 100 parts by weight which is the total of the components $\underline{a}$ and $\underline{b}$.

4. A gasket according to claims 1 to 3, wherein the components $\underline{a}$ and $\underline{b}$ are arranged to be respectively 35 to 60 wt% and 65 to 40 wt% with respect to 100 parts by weight which is the total of the components $\underline{a}$ and $\underline{b}$.

5. A medical device including a cylindrical body and a gasket capable of moving in close contact with the inner surface of said cylindrical body, said medical device being characterized in that said gasket is the type according to either Claims 1 to 4.

**Patentansprüche**

1. Dichtung, bestehend im wesentlichen aus einer Zusammensetzung umfassend einen Abkömmling mit Wasserstoffzusatz (Komponente a) eines Styrol-Butadien-Blockcopolymers, ausgedrückt durch die allgemeine Formel $A\text{-}(B\text{-}A)_n$ oder $A\text{-}B\text{-}(A\text{-}B)_n$, wobei A einen Polymerblock aus monovinyl-substituiertem aromatischem Kohlenwasserstoff darstellt, B einen konjugierten Dienelastomer-Copolymerblock darstellt und n eine ganze Zahl im Bereich zwischen 1 und 5 darstellt, Weichmacher für Kautschuk (Komponente b) und Polyesterelastomer (Komponente c), wobei die Komponente so festgesetzt ist, daß sie 20 bis 150 Gewichtsteile in bezug auf 100 Gewichtsteile ist, die die Summe der Komponenten a und b sind.

2. Dichtung nach Anspruch 1, wobei die Zusammensetzung gebildet ist, indem 20 bis 150 Gewichtsteile der Komponente c und 100 Gewichtsteile, bestehend aus der Komponente a und der Komponente b, gemischt werden, wobei 100 Gewichtsteile die Summe der Komponente a bei 30 bis 70 Gew% und der Komponente b bei 70 bis 30 Gew% sind.

3. Dichtung nach Anspruch 1 oder 2, wobei die Komponente c so festgesetzt ist, daß sie 30 bis 100 Gewichtsteile in bezug auf 100 Gewichtsteile ist, die die Summe der Komponenten a und b sind.

4. Dichtung nach den Ansprüchen 1 bis 3, wobei die Komponenten a und b so festgesetzt sind, daß sie jeweils 35 bis 60 Gew% und 65 bis 40 Gew% in bezug auf 100 Gewichtsteile sind, die die Summe der Komponenten a und b sind.

5. Medizinische Vorrichtung, umfassend einen zylindrischen Körper und eine Dichtung, die in der Lage ist, sich in engem Kontakt mit der Innenfläche des zylindrischen Körpers zu bewegen, wobei die medizinische Vorrichtung dadurch **gekennzeichnet** ist, daß die Dichtung der Typ nach einem der Ansprüche 1 bis 4 ist.


**Revendications**

1. Joint d'étanchéité, constitué essentiellement d'une composition comprenant un dérivé hydrogéné (composant a) d'un copolymère séquencé styrène/butadiène, représenté par la formule générale $A\text{-}(B\text{-}A)_n$ ou $A\text{-}B\text{-}(A\text{-}B)_n$, dans laquelle A représente une séquence polymère d'un hydrocarbure aromatique monovinylique, B représente une séquence copolymère élastomère de diène conjugué, et n représente un nombre entier valant entre 1 et 5, un plastifiant pour caoutchouc (composant b) et un élastomère polyester (composant c), la quantité de composant c représentant de 20 à 150 parties en poids, pour 100 parties en poids du total des composants a et b.

2. Joint d'étanchéité conforme à la revendication 1, dans lequel ladite composition est formée par mélange de 20 à 150 parties en poids dudit composant c et de 100 parties en poids desdits composants a et b, ces 100 parties représentant le total de 30 à 70 % en poids de composant a et de 70 à 30 % en poids de composant b.

3. Joint d'étanchéité conforme à la revendication 1 ou 2, dans lequel la quantité de composant c représente de 30 à 100 parties en poids pour 100 parties en poids du total des composants a et b.

4. Joint d'étanchéité conforme à l'une des revendications 1 à 3, dans lequel les quantités de composants a et b valent respectivement de 35 à 60 % en poids et de 65 à 40 % en poids, pour 100 parties en poids du total des composants a et b.

5. Dispositif médical comprenant un corps cylindrique et un joint d'étanchéité capable de se déplacer en restant en contact étroit avec la surface interne dudit corps cylindrique, ce dispositif médical étant caractérisé en ce que ledit joint d'étanchéité est du type conforme à l'une des revendications 1 à 4.

# FIG.1

# FIG.2(a)

# FIG.2(b)

EMBODIMENT 1

START 10min. 20min. 30min. 40min. 50min. 60min.

EMBODIMENT 2

EMBODIMENT 3

COMPARATIVE EXAMPLE 4